# EUROPEAN PATENT SPECIFICATION

(11) **EP 3 268 026 B1**
(45) Date of publication and mention of the grant of the patent: **04.03.2020**
(21) Application number: 16762691.0
(22) Date of filing: 14.03.2016
(51) Int. Cl.: A61K 45/06, A61K 31/138, C07K 16/28, A61K 39/00, A61K 38/17, A61K 39/04, A61K 39/39, A61K 39/395

(54) **COMBINATION OF ADRENERGIC RECEPTOR ANTAGONISTS AND CHECK POINT INHIBITORS FOR IMPROVED EFFICACY AGAINST CANCER**
KOMBINATION AUS ADRENERGEN REZEPTORANTAGONISTEN UND KONTROLLPUNKTHEMMERN ZUR VERBESSERTEN WIRKSAMKEIT GEGEN KREBS
COMBINAISON D'ANTAGONISTES DE RÉCEPTEUR SS-ADRÉNERGIQUE ET D'INHIBITEURS DE POINT DE CONTRÔLE POUR AMÉLIORER L'EFFICACITÉ CONTRE LE CANCER

(30) Priority: 12.03.2015 US 201562132286 P
(43) Date of publication of application: 17.01.2018
(73) Proprietor: Health Research, Inc., Buffalo NY 14263 (US)
(72) Inventor: REPASKY, Elizabeth, Buffalo, NY 14263 (US); HYLANDER, Bonnie, Buffalo, NY 14263 (US)
(74) Representative: FRKelly
(86) International application number: PCT/US2016/022277
(87) International publication number: WO 2016/145427

(56) References cited:
- WO-A1-2013/173223
- WO-A1-2013/173223
- WO-A1-2014/039983
- WO-A2-2015/013388
- US-B1- 8 945 560
- US-B1- 9 045 545
- KIM-FUCHS C. ET AL: "Chronic stress accelerates pancreatic cancer growth and invasion: a critical role for beta-adrenergic signaling in the pancreatic microenvironment.", BRAIN BEHAV. & IMMUN., vol. 40, 2014, pages 40-47, XP002783683,
- S. W. COLE ET AL: "Molecular Pathways: Beta-Adrenergic Signaling in Cancer", CLINICAL CANCER RESEARCH, vol. 18, no. 5, 20 December 2011 (2011-12-20), pages 1201-1206, XP055310426, US ISSN: 1078-0432, DOI: 10.1158/1078-0432.CCR-11-0641
- TANG J. ET AL.: "Beta adrenergic system, a backstage manipulator regulating tumour progression and drug target in cancer therapy.", SEMINARS IN CANCER BIOLOGY, vol. 23, no. 6, Part B, December 2013 (2013-12), pages 533-542, XP028777328, DOI: 10.1016/j.semcancer.2013.08.009
- COLE ET AL.: 'Molecular Pathways: Beta-Andrenergic Signaling in Cancer''.' CLIN CANCER RES vol. 18, no. 5, 20 December 2011, pages 1201 - 1206., XP055310426
- CHAWLA A. ET AL.: "Immune checkpoints: A therapeutic target in triple negative breast cancer.", ONCOIMMUNOLOGY, vol. 3, E28325, April 2014 (2014-04),

## Description

### FIELD

The present disclosure relates generally to anti-cancer approaches and more specifically to combinations of β-adrenergic receptor antagonists and checkpoint inhibitors.

### BACKGROUND

There is much evidence that patients are able to mount an immune response against their own tumors, however tumors often remain unharmed because of their ability to actively suppress, or even kill, immune cells. There are many approaches being developed to overcome this suppression and develop effective immunotherapies for cancer patients. Among the most promising are vaccines and adoptive T-cell transfer. However, these therapies are also vulnerable to tumor mechanisms of immunosuppression. Recently, it has been found that tumors employ strategies which mimic the natural mechanisms by which the immune response is downregulated, specifically by expression of so called immune checkpoint ligands which interact with receptors on immune cells to block their activity. "Immune checkpoint inhibitors" have been developed to block these interactions. One such example of these inhibitors are antibodies to PD-1 (programmed cell death-1), a receptor on the T-cells which when bound by PD-L1 (a molecule often expressed on tumor cells) inhibits the activity of the T-cells and suppresses the anti-tumor immune response. However, there is an ongoing need for compositions and methods that can enhance the efficacy of check point inhibitors. The present disclosure relates to this need.

WO2013173223 discloses a method for immunotherapy of a cancer patient, comprises administering to the patient an Ab that inhibits signaling from the PD-1/PD-L1 signaling pathway, or a combination of such Ab and an anti-CTLA-4 Ab.

Kim-Fuchs et al. (Brain Behav Immun. 40:40-47) used in vivo optical imaging to non-invasively track growth and dissemination of primary pancreatic cancer using an orthotopic mouse model. Stress-induced neural activation increased primary tumor growth and tumor cell dissemination to normal adjacent pancreas. These effects were associated with increased expression of invasion genes by tumor cells and pancreatic stromal cells.

Cole & Sood (Clin Cancer Res. 18(5):1201-6) describes that beta-adrenergic signaling has been found to regulate multiple cellular processes that contribute to the initiation and progression of cancer, including inflammation, angiogenesis, apoptosis/anoikis, cell motility and trafficking, activation of tumor-associated viruses, DNA damage repair, cellular immune response, and epithelial-mesenchymal transition.

Tang et al. (Seminars in Cancer Biology, 23B: 533-542) summarize recent advancements about the influence of stress hormones, nicotine and β-adrenoceptors on cancer cell proliferation, apoptosis, invasion and metastasis, and also tumour vasculature normalization.

Chawla et al. (Oncoimmunology, 3(3):e28325) demonstrated PD-L1 expression in 20% of triple negative breast cancers suggesting that targeting the PD-1/PD-L1 immune checkpoint may be an effective treatment modality in patients with this disease.

### SUMMARY

The invention relates to the treatment of cancer. Specifically, the invention is defined in the appended claims. It concerns Propanolol and an immune checkpoint inhibitor for use in a method of therapy of cancer in an individual having the cancer, wherein: a) the cancer is selected from breast cancer and melanoma; b) the immune checkpoint is selected from an anti-PD-1 antibody or a PD-1 binding fragment thereof; and c) the propanolol and the immune checkpoint synergistically inhibit cancer progression, and/or inhibit increase in the tumor volume, and/or reduce tumor volume, and/or reduce tumor growth rate, and/or eradicate the tumor or tumor cells. The invention as claimed further concerns such medical uses especially in the event of resistance to the checkpoint inhibitor in the absence of propanolol.

Beyond the invention defined in the appended claims, the present disclosure relates generally to non-selective β-blockers and an immune checkpoint inhibitors for use in methods for therapy of cancer. The use comprises administering to an individual in need thereof an effective amount of a β-blocker and an immune checkpoint inhibitor such that growth of cancer in the individual is inhibited.

In some cases, the individual may be diagnosed as, or suspected of having, or developing a cancer that is resistant to an immune checkpoint inhibitor. In other cases the individual might not be treated, and/or may not have previously been treated with a β-blocker. In yet other cases the individual may have a cancer that exhibits resistance to treatment with a checkpoint inhibitor, wherein the individual is not treated with a β-blocker when the resistance is exhibited, and administering to the individual the checkpoint inhibitor and the β-blocker such that growth of the cancer is inhibited.

Methods can be performed using any suitable checkpoint inhibitors and β-blockers, and can include using more than one of either of these types of agents. In particular and non-limiting examples the immune checkpoint inhibitor is selected from anti-programmed cell death protein 1 (PD-1) antibody or PD-1 binding fragment thereof, or an anti-PD-1 principal ligand (PD-L1) antibody or PD-L1 binding fragment thereof. PD-1 and PD-L1 are well characterized in the art. As claimed herein, the immune checkpoint inhibitor is selected from anti-programmed cell death protein 1 (PD-1) antibody or a PD-1 binding fragment thereof.

The β-blocker may be a nonselective β-blocker and thus be pertinent to the three presently known types of beta receptors (β1, β2 and β3 receptors). It may interfere with one or more of these receptors binding to their endogenous ligands and thus may be competitive antagonists for any β-adrenergic receptor(s). For the purposes of the invention, as claimed herein, the β-blocker is propranolol.

Preferably, administering the β-blocker and the immune checkpoint inhibitor results in a greater than additive inhibition of growth of the cancer, viz, the combination has a synergistic effect.

In certain cases the individual treated with a combination of this disclosure may have a cancer that was treated with the checkpoint inhibitor without the β-blocker, wherein the cancer was resistant to the checkpoint inhibitor, and wherein the β-blocker and the immune checkpoint inhibitor are subsequently administered to the individual such that growth of the cancer is inhibited.

Conversely, the individual may have a cancer that exhibits resistance to treatment with a checkpoint inhibitor, wherein the individual is not treated with a β-blocker when the resistance is exhibited, and administering to the individual the checkpoint inhibitor and the β-blocker such that growth of the cancer is inhibited.

### BRIEF DESCRIPTION OF FIGURES

Figure 1. Graph of data obtained using Pan02 pancreatic cell line tumors in C57BL/6 mice. Treatment of the mice with the β-blocker propranolol significantly improved the efficacy of anti-PD-1 checkpoint inhibition. *p<0.05.
Figure 2. Graph of data demonstrating that β-AR blockade improves the efficacy of anti-PD-1 immunotherapy in a breast cancer model. Data depict absolute tumor volume. Neither propranolol nor anti-PD-1 mAb alone has any impact on tumor growth. But a combination of β-AR antagonist and checkpoint inhibitor results in a greater than additive reduction in absolute tumor volume growth.
Figure 3. Graph of data demonstrating that β-AR blockade improves the efficacy of anti-PD-1 immunotherapy in a breast cancer model. Data depict relative tumor volume.
Figures 4A and 4B represent data obtained in a melanoma model. Individual tumor growth curves (Fig. 4A) and tumor growth rates (Fig. 44B) show a combination of anti-PD-1 and β-blocker is effective in melanoma tumors that do not respond to anti-PD-1 as a monotherapy.

### DETAILED DESCRIPTION

The invention relates to the treatment of cancer. Specifically, the invention is defined in the appended claims. It concerns Propanolol and an immune checkpoint inhibitor for use in a method of therapy of cancer in an individual having the cancer, wherein: a) the cancer is selected from breast cancer and melanoma; b) the immune checkpoint is selected from an anti-PD-1 antibody or a PD-1 binding fragment thereof; and c) the propanolol and the immune checkpoint synergistically inhibit cancer progression, and/or inhibit increase in the tumor volume, and/or reduce tumor volume, and/or reduce tumor growth rate, and/or eradicate the tumor or tumor cells. The invention as claimed further concerns such medical uses especially in the event of resistance to the checkpoint inhibitor in the absence of propanolol.

Beyond the invention defined in the appended claims, the disclosure generally relates to non-selective β-blockers and an immune checkpoint inhibitors for use in a method for therapy of cancer.

The individual in need of treatment in accordance with this disclosure may be any mammal, including but not limited to a human. The cancer type is not particularly limited, other than being a cancer type for which immune checkpoint inhibition may be a suitable prophylactic and/or therapeutic approach. The patient may be at risk for, is suspected of having, or has been diagnosed with a cancer. The cancer may be lung, colon, breast, pancreatic, brain, liver, bladder, kidney, melanoma, ovary, testicular, esophageal, gastric, fibrosarcoma, rhabdomyosarcoma, head and neck, renal cell, thyroid, or a blood cancer. As claimed herein, the cancer is selected from breast cancer, and melanoma.

The disclosure is also pertinent to approaching cancers that are or may become resistant to treatment with one or more immune checkpoint inhibitors. Thus in certain implementations the individual has been previously treated for cancer with a checkpoint inhibitor, but was not given any β-blockers while being treated with the checkpoint inhibitor, and the cancer was initially resistant, or develops resistance, to the checkpoint inhibitor treatment. The disclosure thus includes selecting an individual who has cancer that is resistant to a checkpoint inhibitor as a monotherapy, and administering to the individual a checkpoint inhibitor and a β-blocker. The individual who is resistant to a checkpoint inhibitor as a monotherapy accordingly means an individual who was administered a checkpoint inhibitor for the cancer, but was not also administered a β-blocker, and the cancer was resistant to the treatment that included the checkpoint inhibitor but not the β-blocker. The monotherapy may have included other anti-cancer agents or other interventions, so long as such other agents did not include the β-blocker that is subsequently used in a combination therapy of this disclosure. In one embodiment the individual who is treated with a combination approach described herein has never been previously treated with a β-blocker. In certain embodiments the individual who is treated with a combination therapy of this disclosure has not been diagnosed with, or is not suspected of having, or is not a risk for developing a non-cancerous condition for which a β-blocker would ordinarily be prescribed.

The combination of an immune checkpoint inhibitor and a β-blocker, as claimed herein, exerts a synergistic effect against cancer, which may comprise but is not limited to a greater than additive inhibition of cancer progression, and/or a greater than additive inhibition of an increase in tumor volume, and/or a reduction in tumor volume, and/or a reduction in tumor growth rate, and/or an eradication of a tumor and/or cancer cells. The method may also result in a prolonging of the survival of the individual.

The disclosure also comprises monitoring the treatment of an individual who is receiving a combination of an immune checkpoint inhibitor and a β-blocker. This approach comprises administering the combination of an immune checkpoint inhibitor and a β-blocker as a cancer treatment, testing the individual and/or a biological sample from the individual to determine the efficacy of the combination therapy, and if determined to be necessary, adjusting the combination therapy by, for example, changing the amount of the immune checkpoint inhibitor or the β-blocker, or both, and/or changing the type of immune checkpoint inhibitor and or the β-blocker. Retesting and changing the combination therapy may also be performed.

As is known in the art, β-blockers comprise a class of drug compounds that are typically used for management of cardiac arrhythmias, inhibition of secondary myocardial infarction, management of hypertension, and other indications. The present disclosure includes using any one or any combination of β-blockers that are selective or non-selective antagonists of any one or any combination of the three presently known types of beta receptors (β1, β2 and β3 receptors), or otherwise interfere with one or more of these receptors binding to their endogenous ligands, i.e., epinephrine and/or other stress hormones. Thus, β-blockers used in this disclosure may comprise a class of competitive antagonists for β-adrenergic receptors. In one disclsoure, the β-blocker is a nonselective β-blocker, such as a sympatholytic β-blocker. As claimed herein, the β-blocker is propranolol. In certain disclosures the β-blocker is selected from Bucindolol, Carteolol, Carvedilol, Labetalol, Nadolol, Oxprenolol, Penbutolol, Pindolol, Sotalol, and Timolol.

The immune checkpoint inhibitor used in combination with the one or more β-blockers can be any immune checkpoint inhibitor. As is known in the art, an example of an immune checkpoint is the transmembrane programmed cell death 1 protein (PDCD1, PD-1; also known as CD279) and its ligand, PD-1 ligand 1 (PD-L1, CD274). In normal, non-malignant physiology, PD-L1 on the surface of a cell binds to PD1 on the surface of an immune cell, which inhibits the activity of the immune cell. PD-L1 up-regulation on cancer cell surfaces is thought to facilitate evasion of the host immune system, at least in part by inhibiting T cells that would otherwise target the tumor cell. In alternative disclosures, other immune checkpoints can be inhibited, such CTLA-4. As claimed herein, the immune checkpoint inhibitor is selected from an anti-PD-1 antibody or a PD-1 binding fragment thereof.

Any one or more checkpoint inhibitors can be combined with any one or more β-blockers for use in the methods of this disclosure. In certain embodiments, the checkpoint inhibitors that are combined with the β-blockers comprise antibodies that bind to PD-1, or anti-PD-L1, such as Nivolumab. In another disclosure, the checkpoint inhibitor is an antibody that targets CTLA-4, such as Ipilimumab. In another disclosure the checkpoint inhibitor is targets CD366 (Tim-3), which is a transmembrane protein also known as T cell immunoglobulin and mucin domain containing protein-3.

In alternative disclosures, the checkpoint inhibitors comprise small molecules or other agents that disrupt the immune checkpoint that is exploited by cancer cells to evade cell-mediated or other immune-mediated targeting.

Those skilled in the art, given the benefit of the present disclosure, will recognize how to determine an effective amount of the combination of checkpoint inhibitor and β-blocker for treatment of cancer. In general, and without intending to be bound by any particular theory, it is expected that the amounts of each of these agents that are used and/or tested currently in humans for their separate indications will also be effective in the presently provided combination approach. But modifications can be made by medical professionals based on known conditions, such as the size, age, gender and overall health profile of the individual, the type and stage of the cancer, and other conditions and risk factors that will be otherwise apparent to those skilled in the art. In embodiments, administering the checkpoint inhibitor and the β-blocker has a greater than additive effect on tumor inhibition, relative to use of either agent alone. A greater than additive effect can be determined by comparing the effects of one or both of the agents to any suitable reference, including but not limited to a predetermined value.

In embodiments, one or more β-blockers and one or more immune checkpoint inhibitors are administered concurrently. In embodiments, the one or more β-blockers and one or more immune checkpoint inhibitors are combined into a single pharmaceutical formulation. In embodiments, the one or more β-blockers and the one or more immune checkpoint inhibitors are administered sequentially. The β-blocker and immune checkpoint inhibitor can be administered via any suitable route, including but not necessarily limited to intravenous, intramuscular, subcutaneous, oral, and parenteral routes.

In an embodiment, the combination therapy has a greater than additive inhibition of tumor growth, which may be determined using any suitable measurement, non-limiting examples of which include determining tumor volume or tumor growth rate. The combination therapy can be combined with any other, conventional cancer therapies, including but not limited to surgical and chemotherapeutic approaches.

The following specific examples are provided to illustrate the invention, but are not intended to be limiting in any way.

### Example 1

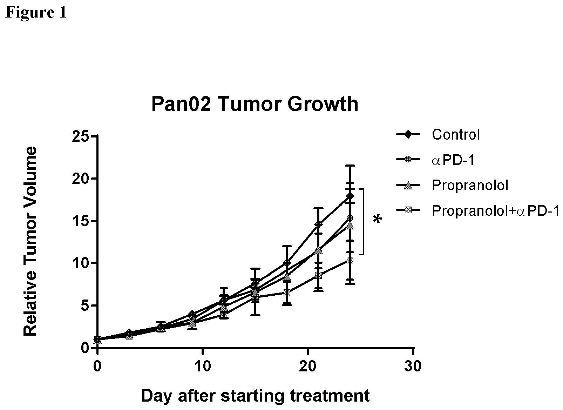

This Example shows that a combination of a β-blocker and an immune checkpoint inhibitor inhibits tumor growth, and demonstrates that the combination is capable of eliciting a greater than additive inhibition of tumor growth. In particular, and without intending to be constrained by any particular, theory, it is considered that combining specific blockade of a stress response with a β-blocker will reverse the systemic immunosuppression caused by stress, such as cancer, and when given in combination with a immune checkpoint inhibitor, will result in both improved activation (i.e., effect of the β-blocker) and sustained activity (i.e., effect of the checkpoint inhibitor) of immune cells and result in significantly improved efficacy of immune cells against the tumor. Accordingly, as shown in Figure 1, β-AR blockade improves the efficacy of anti-PD-1 immunotherapy in a pancreas cancer model. To obtain the data presented in Figure 1, a murine pancreatic tumor (Pan02) was engrafted in C57BL/6 mice. When tumors reached an average size of 50- 100mm³, tumor bearing mice were assigned to one of four experimental groups and treatment was initiated. Groups of mice received: (1) saline (control), (2) anti-PD-1 antibody (BioExcel, 200µg/dose every 3 days), (3) the β-adrenergic signaling antagonist propranolol (10 mg/kg daily by intraperitoneal injection) or (4) combination therapy. Tumor growth was monitored and graphed as relative tumor volume compared to each tumor's starting volume. As can be seen in Figure 1, neither anti-PD-1 nor propranolol alone caused a statistically significant decrease in tumor growth. However, tumor growth was statistically significantly slowed in mice receiving the combination therapy (Students t-test) such that the effect was greater than additive. The data accordingly indicate that the combination therapy can be effective against pancreatic tumors that are resistant to treatment with the checkpoint inhibitor alone.

### Example 2

This Example demonstrates that β-AR blockade improves the efficacy of anti-PD-1 immunotherapy in a breast cancer model. These data, and those obtained using a B16 tumor data provided below were obtained as described for Figure 1, except β-AR blockade (the propranolol treatment) was started three days before administering the anti-PD-1 checkpoint inhibitor, and this 4T1 breast cancer model used BALB/c mice.

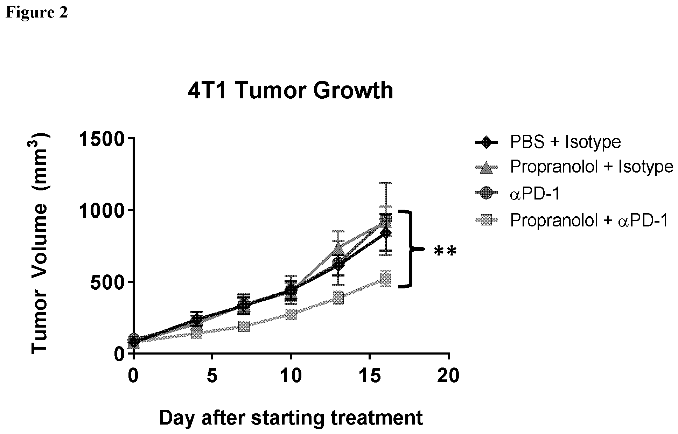

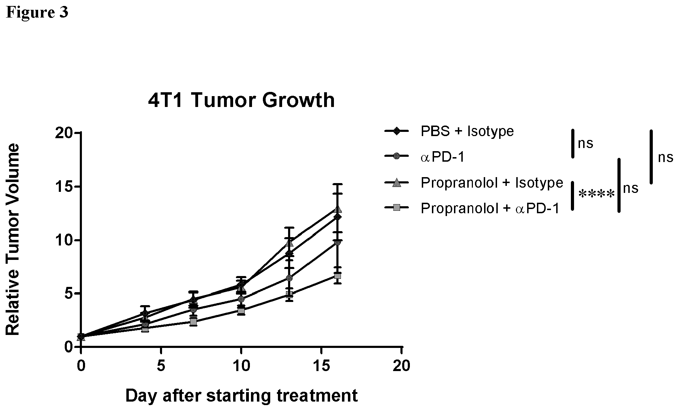

As can be seen from Figures 2 and 3 (measuring absolute tumor volume and relative tumor volume, respectively), neither propranolol nor anti-PD-1 mAb alone has any impact on tumor growth. But a combination of β-AR antagonist and checkpoint inhibitor results in a greater than additive reduction in absolute tumor volume growth. Thus, as with the pancreatic cancer model, the data demonstrate that this combination approach is effective even in breast cancer tumors that do not respond to anti-PD-1 as a monotherapy.

### Example 3

This Example demonstrates that β-AR blockade improves the efficacy of anti-PD-1 immunotherapy in a melanoma model.

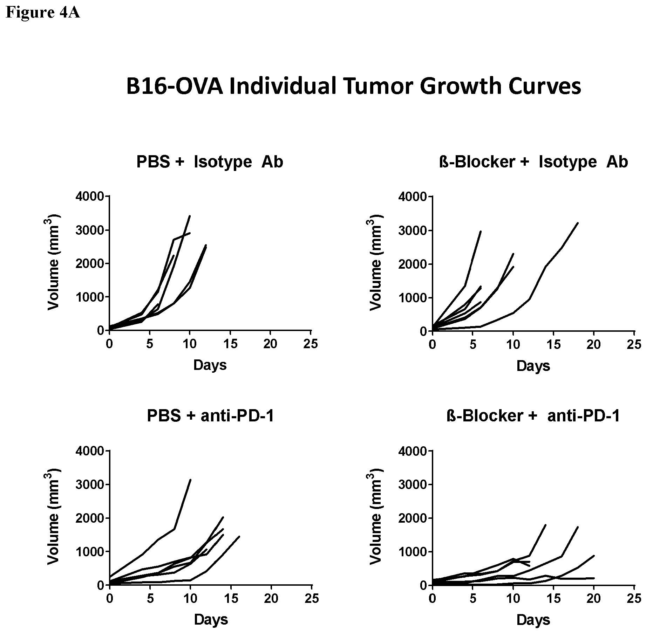

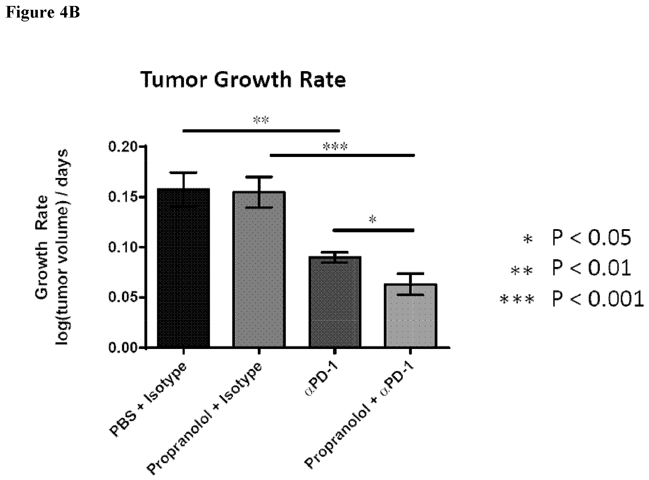

As can be seen from Figures 4A and 4B, which summarize data reflecting individual tumor growth curves (Figure 4A) and tumor growth rates (Figure 4B), the combination approach is effective even in melanoma tumors that do not respond to anti-PD-1 as a monotherapy.

While the invention has been described through specific embodiments, routine modifications will be apparent to those skilled in the art and such modifications are intended to be within the scope of the present invention. But as can be seen from the foregoing examples, and again without intending to be constrained by any particular theory, it is believed that the presently described combination approach is superior to the use of either single agent because the β-blocker will act to reverse the high levels of immunosuppressive cells (MDSC and T-regs) induced by the tumor, thus allowing the activation of the anti-tumor immune response, while the checkpoint inhibitor (shown here with an anti-PD-1 antibody) will bind to the PD-1 molecule (or other checkpoint molecule depending on the inhibitor), which is in this case expressed on the surface of activated immune cells (cytoxic T lymphocytes) and prevent ligation by tumor expressed PD-L1 which would otherwise lead to CTL inhibition. Therefore, this two-pronged approach both allows activation of the immune cells and sustains that activity long enough to support anti-tumor efficacy, even in distinct tumor types that are resistant to at least one checkpoint inhibitor.

## Claims

1. Propranolol and an immune checkpoint inhibitor for use in a method of therapy of cancer in an individual having the cancer, wherein:
a) the cancer is selected from breast cancer and melanoma;
b) the immune checkpoint inhibitor is selected from an anti-PD-1 antibody or a PD-1 binding fragment thereof; and
c) the propranolol and the immune checkpoint inhibitor synergistically inhibit cancer progression, and/or inhibit increase in the tumor volume, and/or reduce tumor volume, and/or reduce tumor growth rate, and/or eradicate the tumor or tumor cells.

2. Propranolol and the immune checkpoint inhibitor for use of claim 1, wherein the cancer exhibits resistance to treatment with the checkpoint inhibitor in the absence of the propranolol.

3. Propranolol and the immune checkpoint inhibitor for use of claim 1 or 2, wherein the individual was previously treated with the immune checkpoint inhibitor but without the propranolol, and wherein the cancer was resistant to the immune checkpoint inhibitor in the absence of the propranolol.

4. Propranolol and the immune checkpoint inhibitor for use of claim 1, wherein the propanolol and the immune checkpoint synergistically inhibit an increase in tumor volume, or inhibit tumor growth rate.

## Patentansprüche

1. Propranolol und ein Immun-Checkpoint-Inhibitor für die Verwendung in einem Verfahren zur Therapie von Krebs bei einem Individuum, das den Krebs hat, wobei:
a) der Krebs aus Brustkrebs und Melanom ausgewählt ist;
b) der Immun-Checkpoint-Inhibitor aus einem Anti-PD-1-Antikörper oder einem PD-1-Bindungsfragment davon ausgewählt ist; und
c) das Propranolol und der Immun-Checkpoint-Inhibitor synergistisch die Krebsprogression inhibieren und/oder die Zunahme des Tumorvolumens inhibieren und/oder das Tumorvolumen reduzieren und/oder die Tumorwachstumsrate reduzieren und/oder den Tumor oder die Tumorzellen beseitigen.

2. Propranolol und der Immun-Checkpoint-Inhibitor für die Verwendung nach Anspruch 1, wobei der Krebs Resistenz gegen eine Behandlung mit dem Checkpoint-Inhibitor bei Abwesenheit des Propranolols zeigt.

3. Propranolol und der Immun-Checkpoint-Inhibitor für die Verwendung nach Anspruch 1 oder 2, wobei das Individuum zuvor mit dem Immun-Checkpoint-Inhibitor, doch ohne das Propranolol behandelt wurde und wobei der Krebs gegen den Immun-Checkpoint-Inhibitor bei Abwesenheit des Propranolols resistent war.

4. Propranolol und der Immun-Checkpoint-Inhibitor für die Verwendung nach Anspruch 1, wobei das Propranolol und der Immun-Checkpoint synergistisch eine Zunahme des Tumorvolumens inhibieren oder die Tumorwachstumsrate inhibieren.

## Revendications

1. Propranolol et inhibiteur de point de contrôle immunitaire destinés à être utilisés dans un procédé de traitement du cancer chez un individu ayant le cancer :
a) le cancer étant choisi parmi le cancer du sein et le mélanome ;
b) l'inhibiteur de point de contrôle immunitaire étant choisi parmi un anticorps anti-PD-1 ou un fragment de liaison PD-1 de celui-ci ; et
c) le propranolol et l'inhibiteur de point de contrôle immunitaire inhibant de manière synergétique la progression cancéreuse, et/ou inhibant l'accroissement du volume tumoral, et/ou réduisant le volume tumoral, et/ou réduisant le taux de croissance tumorale, et/ou éradiquant la tumeur ou les cellules tumorales.

2. Propranolol et inhibiteur de point de contrôle immunitaire destinés à être utilisés selon la revendication 1, le cancer présentant une résistance au traitement avec l'inhibiteur de point de contrôle en l'absence du propranolol.

3. Propranolol et inhibiteur de point de contrôle immunitaire destinés à être utilisés selon la revendication 1 ou la revendication 2, l'individu ayant été précédemment traité avec l'inhibiteur de point de contrôle immunitaire mais sans le propranolol, et le cancer ayant été résistant à l'inhibiteur de point de contrôle immunitaire en l'absence du propranolol.

4. Propranolol et inhibiteur de point de contrôle immunitaire destinés à être utilisés selon la revendication 1, le propranolol et le point de contrôle immunitaire inhibant de manière synergique une augmentation du volume tumoral, ou inhibant le taux de croissance tumorale.
